# EUROPEAN PATENT APPLICATION

(11) **EP 3 498 236 A1**
(43) Date of publication of application: **19.06.2019**
(21) Application number: 16912706.5
(22) Date of filing: 10.08.2016
(51) Int. Cl.: A61F 5/11

(54) **DEVICE FOR CORRECTING NAIL DEFORMITY**

(71) Applicant: Reflepro Japan Co., Ltd., Osaka-shi, Osaka 542-0081 (JP)
(72) Inventor: YOSHINO, Masafumi, Osaka-shi Osaka 542-0081 (JP)
(74) Representative: Zimmermann, Tankred Klaus
(86) International application number: PCT/JP2016/073628
(87) International publication number: WO 2018/029826

(57) **Abstract**

Provided is a nail deformity correction device easily attachable in an effort-saving manner without damage on the device at the time of attachment.

A first straightening member 12 of this device 10 includes a belt part 18 having a serration part 16, and a first latching part 20 disposed at one end of the belt part 18 in a length direction to be latched at one end edge of the nail in a width direction. A second straightening member 14 has a fastening locking part 24 having an insertion hole 22 into which the belt part 18 is inserted to fasten and lock the belt part 18 to the nail, a lock nail part 26 disposed inside the fastening locking part 24 to be locked to the serration part 16 of the belt part 18 inserted into the insertion hole 22 to inhibit the belt part 18 from returning to a direction opposite to its inserting direction, and a second latching part 28 formed to extend from the fastening locking part 24 to the inserting direction of the belt part 18 to be latched at another end edge of the nail in the width direction.

## Description

### Technical Field

The present invention relates to nail deformity correction devices and, in particular, a device for correcting nail deformity such as a so-called ingrown nail of a foot cutting into a flesh part between the nail and a fingertip as it grows so that one and/or the other side edge end of the nail rolls inside.

### Background Art

In a conventional technology as a background of the present invention, a device for nail straightening has been suggested. In a type having at least two hook-shaped force introduction members (1, 1a, 1b) hooked on a lower side of nail edge parts (3a, 3b) and at least one coupling member (2, 2a, 2b), the coupling member (2, 2a, 2b) is flexible and firmly attached to the force introduction members (1, 1a, 1b). I this case, the coupling member (2, 2a, 2b) is shortened to cause a tensile force adjustable in a stepless manner at the force introduction members (1, 1a, 1b). The tensile force acts on the nail edge parts (3a, 3b) to cause bending moment to a nail plate (for example, refer to Patent Literature 1).

In this conventional device for nail straightening, the entirety is formed of a wire material, and a region of the force introduction members (1, 1a, 1b) is formed of a wire material made of spring steel and is elastically deformable. Also, a region of the coupling member (2, 2a, 2b) is formed of a soft wire material capable of molten binding, and is formed so as to be plastically deformable. The force introduction members and the coupling member have different thickness of wire material.

In this conventional technology, an embodiment including a hook-shaped part (1, 1a, 1b, 13, 15) or an eye (7) at an end of the coupling member (2a, 2b) is disclosed. Also in this conventional technology, in order to stabilize the position of the device for nail straightening, that is, to prevent the end of the hook-shaped part (1, 1a, 1b, 13, 15) from being twisted or rotated to come off from the lower side of nail edge part (3a, 3b) when the device is attached to the nail or during treatment, an embodiment including any of a coated part 17, a bent-out part 18, and a molded portion 19 provided on a side part as a member for positional stabilization is disclosed.

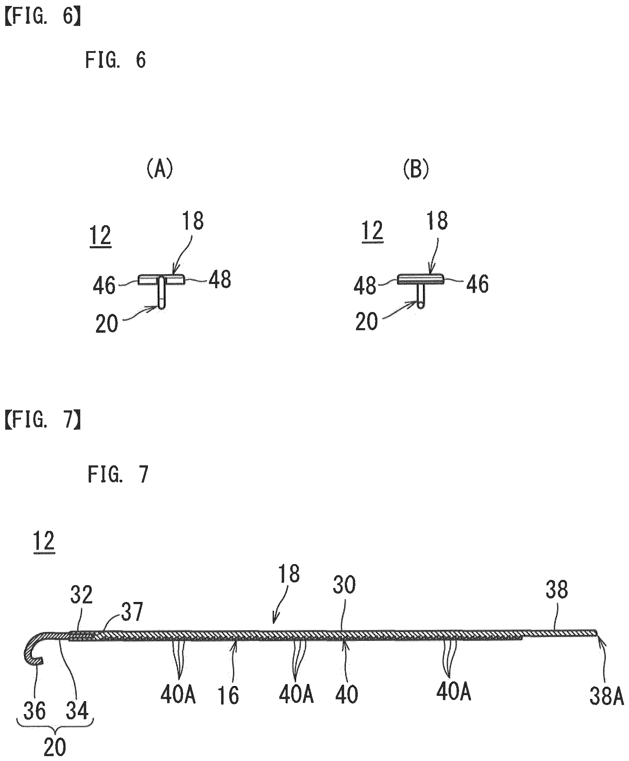

In this conventional technology, securing of the device by an appropriate tool is depicted in FIG. 7. The coupling members (2, 2a, 2b) are guided as being partially wound around a lower pin (12) of the tool, and the eye (7a, 7b) is hooked on an upper pin (11) of the tool. By enlarging a space between the pins (11, 12) both in a stepless manner, that is, consecutively, the space between the hook-shaped members (1a, 1b) both is consecutively shortened until a desired tensile force is achieved. In this manner, in order to establish (fix) the defined space between the hook-shaped members (1a, 1b) both, that is, a defined length of the coupling members, the tool (10) spreads at least 180° in a clockwise direction, thereby making both of the coupling members (2, 2a, 2b) perpetually coupled together. Then, after this device for nail straightening is attached to the nail, an excessive wire material portion of the coupling members (2, 2a, 2b) is cut out by, for example, a cutter.

Also in this conventional technology, fastening the coupling members (2, 2a, 2b) and fixing their lengths may be performed by twining the coupling members (2, 2a, 2b) together.

### Citation List

### Patent Literature

PTL 1: Japanese Unexamined Patent Application No. 2007-289712 (for example, FIG. 1 to FIG. 12)

### Summary of Invention

### Technical Problem

However, in this conventional technology, to fastening the coupling members (2, 2a, 2b), a work of twisting the coupling members by using the dedicated tool is required. Also when a dedicated tool is not used, a work of twining the coupling members (2, 2a, 2b) together is required. Thus, in this conventional technology, burdensome efforts are required to attach the device to the nail, and the attachment time is also long.

Also in this conventional technology, the force introduction members (1, 1a, 1b) and the coupling members (2, 2a, 2b) are coupled as metal wire materials having different degrees of elasticity and different thickness. Thus, when the above-described operation of fastening the coupling members (2, 2a, 2b) and fixing the length is performed, that is, when the work of twisting or twining the coupling members is performed, a boundary portion between a force introduction member (1, 1a, 1b) and a coupling member (2, 2a, 2b) may be broken by bending. Furthermore, when a user changes the shape of a hook in accordance with the thickness and shape of the nail by using special pliers, a boundary portion between a hook portion and any of the above-described members for position stabilization (for example, the coated part 17, the bent-out part 18, and the molded portion 19) may be broken by bending.

Also, in the above-describe conventional technology, when the excessive wire material portion of the coupling members (2, 2a, 2b) is cut out, an edge of its cutout end surface is oriented above the nail. Thus, the hand may be damaged by the edge portion of the cutout end surface. Furthermore, when socks are worn after this device is attached, the edge of the cutout end surface may damage the socks.

Also, in this conventional technology, reversibility is kept before the excessive wire material portion of the coupling members (2, 2a, 2b) is cut out, that is, the nail tensile force can be loosened. However, reversibility is lost after the excessive wire material portion is cut out, that is, it is extremely difficult to loosen the nail tensile force and adjust the tensile force again.

Hence, a main object of the present invention is to provide a nail deformity correction device easily attachable in an effort-saving manner without damage on the device at the time of attachment.

Also, another object of the present invention is to provide a nail deformity correction device in which a straightening force to be applied to the nail can be easily changed even after the device is attached.

### Solution to Problem

The present invention according to claim 1 is directed to a nail deformity correction device including a first straightening member and a second straightening member which correct deformity of a finger nail of a foot in cooperation with each other, the first straightening member including a belt part having a serration part, and a first latching part disposed at one end of the belt part in a length direction to be latched at one end edge of the deformed nail in a width direction, and the second straightening member including a fastening locking part having an insertion hole into which the belt part is inserted to fasten and lock the belt part to the deformed nail, a lock nail part disposed inside the fastening locking part to be locked to the serration part of the belt part inserted into the insertion hole to inhibit the belt part from returning to a direction opposite to an inserting direction of the belt part, and a second latching part formed to extend from the fastening locking part to the inserting direction of the belt part to be latched at another end edge of the deformed nail in the width direction.

Since the present invention according to claim 1 has the above-described structure, the first latching part of the first straightening member is latched at one end edge of the deformed nail in the width direction, the second latching part of the second straightening member is latched at the other end edge of the nail in the width direction. Furthermore, the other end side (free end side) of the belt part in the length direction is inserted into the insertion hole of the lock nail part of the fastening locking part, and the free end side of the belt part is pulled, thereby allowing the belt part to be fastened to the nail surface.

In this case, the one end edge and the other end edge sides of the deformed nail in the width direction are pulled upward by the first latching part and the second latching part, respectively, to a direction opposite to the ingrowing direction of the deformed nail, for example, "ingrown nail". This tensile force can be increased as the length of the belt part to be pulled out from the insertion hole of the fastening locking part is longer, that is, a length between one end part of the belt part in the length direction and a portion of the belt part locked by the lock nail part is shorter. By contrast, this tensile force can be decreased as the length of the belt part to be pulled out from the insertion hole of the fastening locking part is shorter, that is, the length between one end part of the belt part in the length direction and the portion of the belt part locked by the lock nail part is longer. Therefore, this tensile force can be adjusted as appropriate in accordance with the size and shape of the deformed nail, the degree of a curve (the degree of deformation) of the ingrown nail, and so forth.

And, the tensile force adjusted by this adjustment is retained by the lock function of the lock nail part inhibiting the belt part from returning to the direction opposite to the inserting direction of the belt part. Thus, the tensile force to the direction opposite to the ingrowing direction of the ingrown nail continuously acts at both end edges of the ingrown nail in the width direction, and a decrease in this tensile force is prevented. Therefore, according to the nail deformity correction device, deformity of the nail such as ingrown nail can be straightened.

Also as described above, when the belt part is fastened by the fastening locking part, the belt part makes contact with the surface of the nail and pressurizes the nail downward. Thus, in particular, with the belt part as a center, the first latching part, the fastening locking part, and the second latching part are each stably attached to the surface of the nail without being shifted or moved.

Since the present invention has the structure and operations described above, compared with the conventional technology described in Patent Literature 1, the work of twisting coupling members (2, 2a, 2b) by using the dedicated tool or the work of twining the coupling members (2, 2a, 2b) when not using the dedicated tool is not required. Thus, in the nail deformity correction device of the present invention, burdensome efforts to attach this device to the nail are not required, and the attachment time is also short. Also in the conventional technology, when the work of twisting or twining the coupling members is performed, a boundary portion between a force introduction member (1, 1a, and 1b) and a coupling member (2, 2a, 2b) may be broken by bending. By contrast, in the present invention, these twisting work and twining work are not required, and thus inconveniences such as breakage of the device itself by bending are eliminated.

That is, according to the present invention, it is possible to provide a nail deformity correction device easily attachable in an effort-saving manner without damage on the device at the time of attachment, capable of causing an appropriate straightening force to act over a long period, and capable of causing a straightening force to act as intended.

The present invention according to claim 2 is an invention dependent from the invention according to claim 1, and is directed to the nail deformity correction device in which the belt part further includes a belt part main body, a first support part disposed at one end of the belt part main body in a length direction to support the first latching part, and a lead part disposed at another end of the belt part main body in the length direction and having a free end at a tip, to guide the belt part to the second straightening member, and the serration part is disposed between the first support part and the lead part and at an intermediate part on both or either one of main surfaces including one main surface and another main surface of the belt part main body in the length direction, and the serration part is disposed so as to be opposed to the deformed nail surface when the first latching part is latched at one end edge of the deformed nail in the width direction.

Since the present invention according to claim 2 has the above-described structure, in addition to the operations and effects of the invention according to claim 1, the first support part can stably support the first latching part to the nail surface and, furthermore, the lead part can easily guide the belt part to the second straightening part. Also, since the serration part is disposed so as to be opposed to the deformed nail surface, when the nail deformity correction device of the present invention is attached to the deformed nail, the serration part makes contact with the deformed nail surface. In this case, the serration part exerts a shift-preventing function of preventing a positional shift due to slipping of the belt part on the nail surface.

The present invention according to claim 3 is an invention dependent from the invention according to claim 1 or claim 2, and is directed to the nail deformity correction device in which the serration part includes a sawtooth part having an upright surface extending at a substantially right angle with respect to the one main surface and the other main surface of the belt part and a tilted surface tilted toward the inserting direction of the belt part, and the lock nail part includes a nail piece having an upright engaging surface to be engaged with the upright surface of the sawtooth part and a tilted engaging surface to be engaged with the tilted surface of the sawtooth part.

Since the present invention according to claim 3 has the above-described structure, the upright surface of the sawtooth part and the upright engaging surface of the lock nail part can be engaged together, and the tilted surface of the sawtooth part and the tilted engaging surface of the lock nail part can be engaged together. Thus, when the belt part is inserted into the insertion hole of the fastening locking part, the tilted engaging surface of the lock nail part is pressurized by the tilted surface of the sawtooth part to cause the belt part to move to the inserting direction. Also, when the belt part is about to return to the direction opposite to the inserting direction of the belt part, the upright surface of the sawtooth part and the upright engaging surface of the lock nail part are engaged, and a surface contact is made with that engaging area. Thus, the lock function against the return force applied to the belt part is firmly exerted.

The present invention according to claim 4 is an invention dependent from the invention according to any one of claim 1 to claim 3, and is directed to the nail deformity correction device in which the lock nail part is disposed inside the fastening locking part via a cantilever-like movable piece.

Since the present invention according to claim 4 has the above-described structure, one end side of the lock nail part serves as a fixed end supported to the inside of the fastening locking part, and the other end thereof serves as a free end to become in a movable state. If the present invention is the invention dependent from claim 3, when the belt part is inserted into the insertion hole of the fastening locking part, the tilted engaging surface of the lock nail part is pressurized as sliding over the tilted surface of the sawtooth part to be warped to the inserting direction of the belt part. Thus, the belt part can move through the insertion hole to the inserting direction. On the other hand, when the belt part is tried to return to the direction opposite to the inserting direction of the belt part, the upright surface of the sawtooth part and the upright engaging surface of the lock nail part are engaged, and a resistance force acts against the return force of the belt part, thereby locking the return of the belt part.

The present invention according to claim 5 is an invention dependent from the invention according to any one of claim 1 to claim 4, and is directed to the nail deformity correction device in which the movable piece includes a hinge part, and the lock nail part is supported so as to freely rock to a direction orthogonal to the inserting direction of the belt part.

Since the present invention according to claim 5 has the above-described structure, the lock nail part is disposed inside the fastening locking part via the hinge part as a cantilever-like movable piece. Thus, the present invention has operations and effects similar to those of the invention according to claim 4.

The present invention according to claim 6 is an invention dependent from the invention according to any one of claim 3 to claim 5, and is directed to the nail deformity correction device in which, when an angle at which the upright surface and the tilted surface of the sawtooth part cross is taken as θ_{B}, the θ_{B} is set so that 45°<θ_{B}<60°, when an angle at which the upright engaging surface and the tilted engaging surface of the lock nail part cross is taken as θ_{L}, the θ_{L} is set so that 45°<θ_{L}<60°, and, furthermore, it is set so that the θ_{B}=the θ_{L}.

Since the present invention according to claim 6 has the above-described structure, when the belt part is inserted into the insertion hole of the fastening locking part, a tilted angle between the tilted surface of the sawtooth part and the tilted engaging surface of the lock nail part is a tilted angle suitable for the belt part to move to the inserting direction. Thus, the belt part can easily move to the fastening direction via the insertion hole of the fastening locking part and, in turn, fastening of the belt part by the fastening locking part onto the nail surface is facilitated.

The present invention according to claim 7 is an invention dependent from the invention according to any one of claim 1 or claim 2, and is directed to the nail deformity correction device in which the fastening locking part further includes an activation piece connected to the lock nail part and exposed to outside the fastening locking part, and the activation piece releases a locked state between the serration part and the lock nail part by making the activation piece movable in a direction orthogonal to the inserting direction of the belt part and releasing locking between the serration part and the lock nail part.

Since the present invention according to claim 7 has the above-described structure, by making the activation piece movable in the direction of releasing locking between the serration part and the lock nail part, a locked state between the serration part and the lock nail part can be released. That is, the belt part can be moved to a direction opposite to the inserting direction of the belt part. Thus, according to the present invention, even after the device of the present invention is attached to the nail surface, the belt part is moved as appropriate from the insertion hole of the fastening locking part to the direction opposite to the inserting direction to once loosen fastening of the belt part and adjust the strength of fastening, thereby allowing the straightening force applied to the nail surface to be freely and easily changed. Furthermore, according to the present invention, the belt part can be pulled out from the insertion hole of the fastening locking part for reuse of the first straightening member once used.

The present invention according to claim 8 is an invention dependent from the invention according to any one of claim 3 to claim 6, and is directed to the nail deformity correction device in which the fastening locking part further includes an activation piece connected to the lock nail part and exposed to outside the fastening locking part, and the activation piece releases an engaged state between the upright surface of the sawtooth part and the upright engaging surface of the lock nail part and an engaged state between the tilted surface of the sawtooth part and the engaging tilted surface of the lock nail part by making the activation piece movable in a direction orthogonal to the inserting direction of the belt part and isolating an engagement between the sawtooth part and the lock nail part.

Since the present invention according to claim 8 has the above-described structure, by making the activation piece movable, the engaged state between the upright surface of the sawtooth part and the upright engaging surface of the lock nail part and the engaged state between the tilted surface of the sawtooth part and the engaging tilted surface of the lock nail part can be released. Thus, also in the present invention, operations and effects similar to those of the invention according to claim 7 can be exerted.

The present invention according to claim 9 is an invention dependent from the invention according to any one of claim 1 to claim 8, and is directed to the nail deformity correction device in which side wall parts are disposed on both side surface of the belt part in a width direction along the length direction of the belt part where at least the serration part is disposed, and a constricted part constricted to the width direction of the belt part is formed partially on each of the side wall parts so as to be opposed to each other in the width direction of the belt part.

Since the present invention according to claim 9 has the above-described structure, in the present invention, even if a thin portion with a decreased strength is formed on the belt part due to disposition of the serration part, the side wall parts can solve the inconvenience of this decrease in strength. Also, the constricted parts have a function as nipping parts for nipping the belt part when the belt part is inserted into the insertion hole of the fastening locking part, thereby allowing the belt part to be easily inserted into the insertion hole of the fastening locking part.

The present invention according to claim 10 is an invention dependent from the invention according to any one of claim 2 to claim 9, and is directed to the nail deformity correction device in which the lead part of the belt part is formed so as to be in a tapered shape and have a thin cross section.

Since the present invention according to claim 10 has the above-described structure, when the lead part is inserted into the insertion hole of the fastening locking part, the lead part can easily guide the belt part to the insertion hole of the fastening locking part without being interfered by the lock nail part of the fastening locking part.

The present invention according to claim 11 is an invention dependent from the invention according to any one of claim 2 to claim 10, and is directed to the nail deformity correction device in which the first latching part includes a first insert part supported by the first support part, the fastening locking part further includes a second support part provided to extend from an end edge part of the fastening locking part on an exit side of the insertion hole to the inserting direction of the belt part, and the second latching part includes a second insert part supported by the second support part.

Since the present invention according to claim 11 has the above-described structure, the first latching part is firmly supported by the first insert part to the first support part, and the second latching part is firmly supported by the second insert part to the second support part. Thus, the first latching part and the second latching part each have a stable latching function.

The present invention according to claim 12 is an invention dependent from the invention according to any one of claim 1 to claim 11, and is directed to the nail deformity correction device in which the belt part and at least the lock nail part of the fastening locking part of the second straightening member are formed of a flexible plastic material, and the first latching part and the second latching part are each formed of an elastic metal material.

Since the present invention according to claim 12 has the above-described structure, the operations and effects of the present invention according to claims 1 to 11 can be more effectively achieved.

### Advantageous Effects of Invention

According to the nail deformity correction device of the present invention, a main effect can be achieved in which the device can be easily attached in an effort-saving manner without damage on the device at the time of attachment. In this case, the effect of the present invention is achieved in which an appropriate straightening force can be caused to act over a long period and a user-intended straightening force can be caused to act. Also, in addition to the main effect, another effect is also achieved in which the straightening force to be applied to the nail can be easily changed even after the device is attached.

The above-described objects and other objects, features, and advantages of the present invention will become more apparent from the following description of embodiments for carrying out the present invention made with reference to the drawings.

### Brief Description of Drawings

FIG. 1 is a perspective view depicting one example of an embodiment of a first straightening member and a second straightening member of a nail deformity correction device according to the present invention.
FIG. 2 is a perspective view of the first straightening member and the second straightening member of the nail deformity correction device depicted in FIG. 1 when viewed from a bottom side.
FIG. 3 is a plan view depicting one example of the first straightening member depicted in FIG. 1 and FIG. 2.
FIG. 4 is a front view of the first straightening member depicted in FIG. 3.
FIG. 5 is a bottom view of the first straightening member depicted in FIG. 3.
FIG. 6(A) is a left side view of the first straightening member depicted in FIG. 3 and FIG. 6(B) is a right side view of the first straightening member depicted in FIG. 3.
FIG. 7 is a sectional view along a line A-A of FIG. 5.
FIG. 8 is a perspective view depicting one example of the second straightening member of the nail deformity correction device depicted in FIG. 1 and FIG. 2.
FIG. 9 is a plan view of the second straightening member depicted in FIG. 8.
FIG. 10 is a bottom view of the second straightening member depicted in FIG. 8.
FIG. 11 is a front view of the second straightening member depicted in FIG. 8.
FIG. 12 is a right side view of the second straightening member depicted in FIG. 8.
FIG. 13 is a sectional view along a line B-B of FIG. 9.
FIG. 14 is a perspective view depicting one example of a second insert part of a second latching part depicted in FIG. 8.
FIG. 15 is a plan view depicting a state in which the first straightening member is inserted into the second straightening member.
FIG. 16 is a front view of the state depicted in FIG. 15.
FIG. 17 is a bottom view of the state depicted in FIG. 15.
FIG. 18 is a left side view of the state depicted in FIG. 15.
FIG. 19 is a sectional view along a line C-C of FIG. 17.
FIG. 20(A) is an enlarged view of a D part of FIG. 19 and FIG. 20(B) is an enlarged view of main parts of FIG. 20(A).
FIG. 21 is a perspective view depicting one example of a process of attaching the nail deformity correction device depicted in FIG. 1 and FIG. 2 to a deformed nail, in which, for example, the first latching part of the first straightening member is latched at one end edge of a nail in a width direction and the second latching part of the second straightening member is latched at the other end edge of the nail in the width direction.
FIG. 22 is a perspective view depicting a process of, after the process depicted in FIG. 21, inserting a belt part of the first straightening member is inserted into an insertion hole of a fastening locking part of the second straightening member and the belt part is locked by the fastening locking part at a position of being fastened to the deformed nail.
FIG. 23 is a perspective view depicting a process of, after the process depicted in FIG. 22, cutting out a superfluous portion of the belt part protruding from the insertion hole of the fastening locking part at an appropriate location by a cutting tool.
FIG. 24 is a perspective view depicting a process of, after the process depicted in FIG. 23, applying a coating material such as an ultraviolet curable resin material so as to cover a location where the superfluous portion of the belt part is cut out and a root side of the fastening locking part and the second latching part to form an application part.
FIG. 25 is a perspective view depicting a process of, after the process depicted in FIG. 24, irradiating the application part with ultraviolet rays by an ultraviolet LED or the like to cure the coating material.
FIG. 26 is a perspective view depicting a state after the process depicted in FIG. 25, in which the application part is formed as a coated part.
FIG. 27 is a perspective view depicting a modification example of the second latching part of the second straightening member that can be applied to the nail deformity correction device according to the present invention.
FIG. 28 is a plan view of the second straightening member depicted in FIG. 27.
FIG. 29 is a perspective view depicting a second insert part of a second latching part depicted in FIG. 27 and FIG. 28.
FIG. 30 depicts another example of the embodiment of the nail deformity correction device according to the present invention, and is a perspective view depicting one example of a process of attaching a first straightening member and a second straightening member of the nail deformity correction device to a deformed nail.
FIG. 31 is perspective view depicting one example of the second straightening member to be applied to the nail deformity correction device depicted in FIG. 30.
FIG. 32(A) is a sectional view depicting a state when the first straightening member is inserted into the second straightening member depicted in FIG. 30 and FIG. 32(B) is an enlarged view of an E part of FIG. 32(A).
FIG. 33 is a sectional view of an operation state of releasing an engaged state between a sawtooth part of the first straightening member and a lock nail part of the second straightening member from the state depicted in FIG. 32.
FIG. 34 depicts a modification example of the second straightening member depicted in FIG. 30 and FIG. 31 and is an enlarged sectional view of main parts depicting a state in which the first straightening member is inserted into the second straightening member.
FIG. 35 depicts still another example of the embodiment of the nail deformity correction device according to the present invention and is a sectional view depicting a state in which the first straightening member of the nail deformity correction device is inserted into the second straightening member.

### Description of Embodiments

The main object of the present invention easily attachable in an effort-saving manner without damage on the device at the time of attachment has been achieved by providing a nail deformity correction device including a first straightening member and a second straightening member which correct deformity of a finger nail of a foot in cooperation with each other, the first straightening member including a belt part having a serration part, and a first latching part disposed at one end of the belt part in a length direction to be latched at one end edge of the deformed nail in a width direction, and the second straightening member including a fastening locking part having an insertion hole into which the belt part is inserted to fasten and lock the belt part to the deformed nail, a lock nail part disposed inside the fastening locking part to be locked to the serration part of the belt part inserted into the insertion hole to inhibit the belt part from returning to a direction opposite to an inserting direction of the belt part, and a second latching part formed to extend from the fastening locking part to the inserting direction of the belt part to be latched at another end edge of the deformed nail in the width direction.

Also, the other object in which the straightening force to be applied to the nail can be easily changed even after the device is attached has been achieved by providing a nail deformity correction device in which the fastening locking part further includes an activation piece connected to the lock nail part and exposed to outside the fastening locking part, and the activation piece releases a locked state between the serration part and the lock nail part by making the activation piece movable in a direction orthogonal to the inserting direction of the belt part and releasing locking between the serration part and the lock nail part.

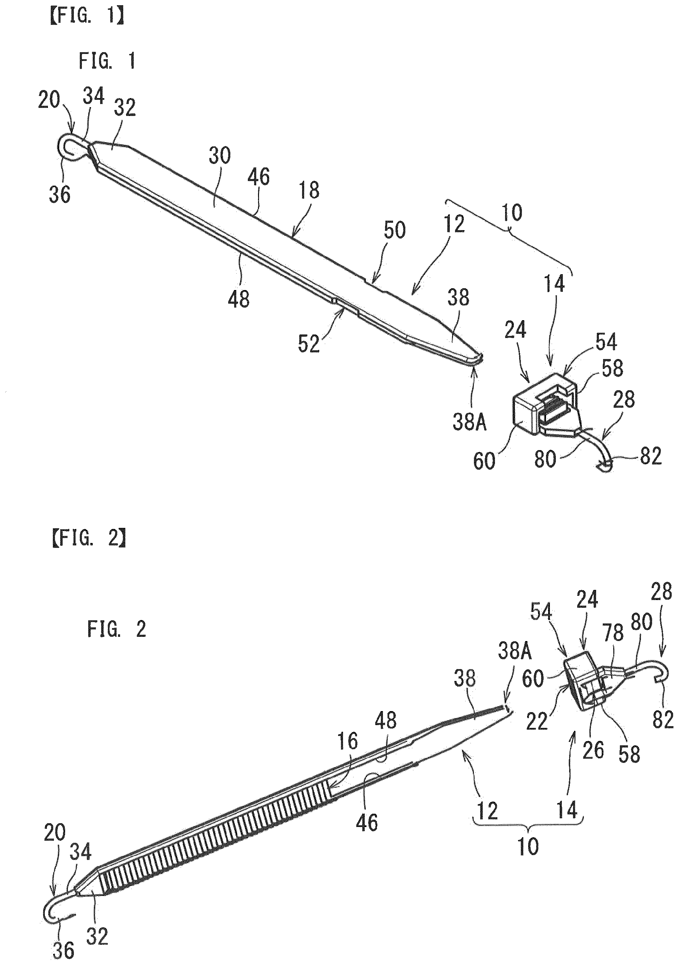

FIG. 1 is a perspective view depicting one example of an embodiment of a first straightening member and a second straightening member of a nail deformity correction device according to the present invention, and FIG. 2 is a perspective view when viewed from a bottom side.

A nail deformity correction device (may be hereinafter simply referred to as a "device" for convenience) 10 according to this embodiment includes, as depicted in FIG. 1 and FIG. 2, a first straightening member 12 and a second straightening member 14 which correct deformity of a finger nail of a foot in cooperation with each other. The first straightening member 12 includes a belt part 18 having a serration part 16 on one main surface (back surface). At one end of the belt part 18 in a length direction, a first latching part 20 to be latched at one end edge of the deformed nail in a width direction is disposed.

The second straightening member 14 includes a fastening locking part 24 having an insertion hole 22 into which the belt part 18 is to be inserted to be fasten and lock the belt part 18 to the deformed nail. Inside the fastening locking part 24, a lock nail part 26 is disposed which inhibits the belt part 18 from returning to a direction opposite to an inserting direction of the belt part 18. The lock nail part 26 is disposed so as to be able to be locked to the serration part 16 of the belt part 18 inserted into the insertion hole 22. Also, the second straightening member 14 includes a second latching part 28, and the second latching part 28 is formed to extend from the fastening locking part 24 to the inserting direction of the belt part 18, and is latched at another end edge of the deformed nail in the width direction.

Here, the first straightening member 12 and the second straightening member 14 are each described in detail.

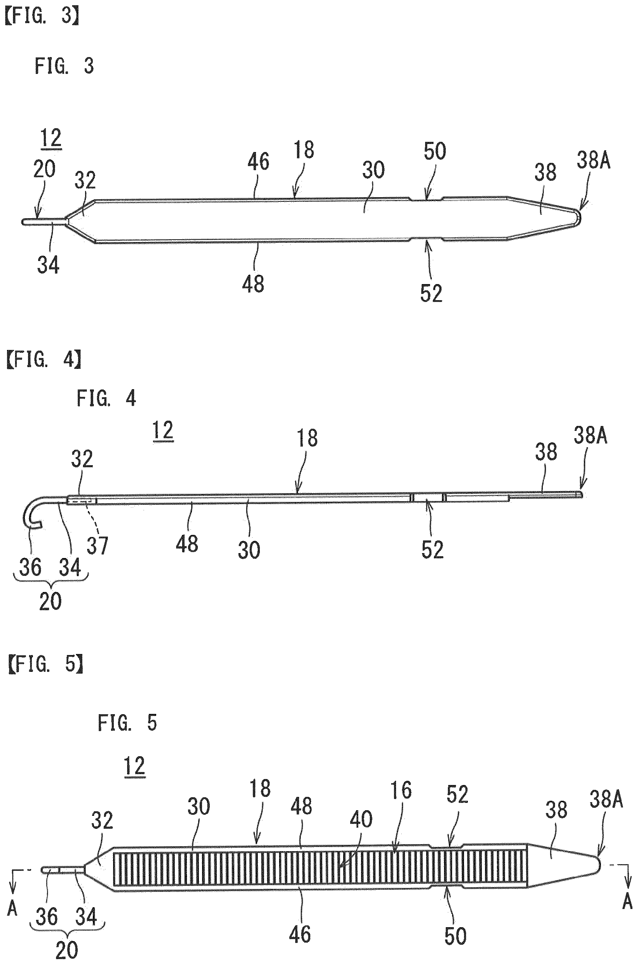

First, the first straightening member 12 is described in detail with reference to, for example, FIG. 3, FIG. 4, FIG. 5, FIG. 6, and FIG. 7. FIG. 3 is a plan view depicting one example of the first straightening member depicted in FIG. 1 and FIG. 2, FIG. 4 is its front view, FIG. 5 is its bottom view, FIG. 6(A) is its left side view, FIG. 6(B) is its right side view, and FIG. 7 is a sectional view along a line A-A of FIG. 5.

The belt part 18 of the first straightening member 12 includes a band-shaped narrowly-elongated belt part main body 30, as depicted in FIG. 3, FIG. 4, and FIG. 5. The belt part main body 30 has a first support part 32 disposed at one end in its length direction. The first support part 32 is formed in a flat plate shape having, for example, a substantially triangular shape in a planar view, to support the first latching part 20 as depicted in FIG. 3 and FIG. 5.

The first latching part 20 includes a stem part 34 as depicted in FIG. 3 and FIG. 5. The stem part 34 has one end side in its axial direction supported by the first support part 32 and has a hook part 36 formed on the other end side in its axial direction as depicted in FIG. 7.

At the other end of the belt part main body 30 in a length direction, a lead part 38 is disposed with its tip as a free end 38A. The lead part 38 is formed in a tapered shape of, for example, a isosceles triangle, in a planar view, as depicted in FIG. 3 and FIG. 5. Furthermore, the lead part 38 is formed having a sectional shape with a material thickness thinner than the thickness of the lead part main body 30, as depicted in FIG. 4 and FIG. 7.

The serration part 16 is disposed between the first support part 32 and the lead part 38 at an intermediate part of one main surface (lower surface) of the belt part main body 30 in the length direction. The serration part 16 is disposed so as to be opposed to the deformed nail surface when the first latching part 20 is latched at one end edge of the deformed nail in the width direction. The serration part 16 may be formed on, in addition to the one main surface (lower surface) of the belt part main body 30, the other main surface (upper surface), and is only required to be formed on a main surface opposed to the nail surface, that is, a main surface in contact with the nail surface.

This serration part 16 is formed of a sawtooth part 40 including, for example, a plurality of consecutive sawtooth pieces 40A. The plurality of sawtooth pieces 40A each have an upright surface 42 extending at a substantially right angle with respect to the one main surface (lower surface) and the other main surface (upper surface) of the belt part main body 30 and a tilted surface 44 tilted toward the inserting direction of the belt part main body 30. When an angle at which the upright surface 42 and the tilted surface 44 of the sawtooth part 40 cross is taken as θ_{B}, θ_{B} is set so as to be in a range of 45°<θ_{B}<60°.

Also, in the belt part 18, a right side wall part 46 and a left side wall part 48 are disposed on one and the other side surfaces, respectively, of the belt part main body 30 in the width direction. These right side wall part 46 and left side wall part 48 on both side surfaces of the belt part main body 30 in the width direction are disposed along the length direction of the belt part main body 30 where the sawtooth part 40 is disposed. These right side wall part 46 and left side wall part 48 are disposed along the length direction of the belt part main body 30 corresponding to at least a portion where the sawtooth part 40 is disposed.

Furthermore, the right side wall part 46 and the left side wall part 48 have, each partially in its length direction, a right-side constricted part 50 and a left-side constricted part 52, respectively, constricted to the width direction of the belt part main body 30. The right-side constricted part 50 and the left-side constricted part 52 are formed so as to be opposed to each other in the width direction of the belt part main body 30.

The belt part main body 30, the first support part 32, the lead part 38, and the sawtooth part 40 of the first straightening member 12 are integrally formed of a flexible material, for example, a plastic material.

Also, the stem part 34 and the hook part 36 of the first latching part 20 are integrally formed of an elastic material, for example, a metal material. Furthermore, the first latching part 20 includes a first insert part 37 as depicted in FIG. 7, and the first insert part 37 is insert-molded by, for example, injection molding, so as to be supported by the first support part 32. In this case, on a root part side of the stem part 34 of the first latching part 20, that is, on a circumferential surface of the first insert part 37 to be inserted into the first support part 32, for example, knurls (not depicted) are formed as a rotation-stop and slip-stop structure.

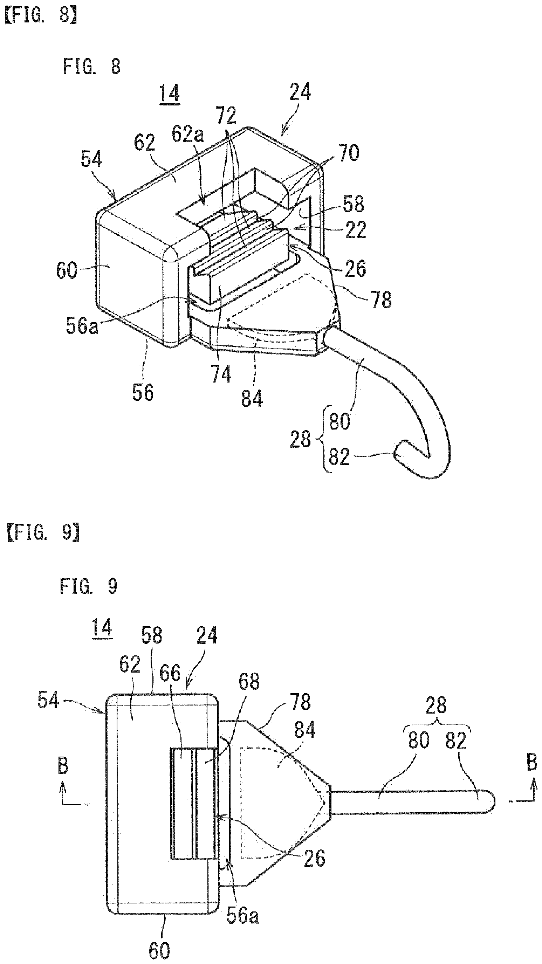

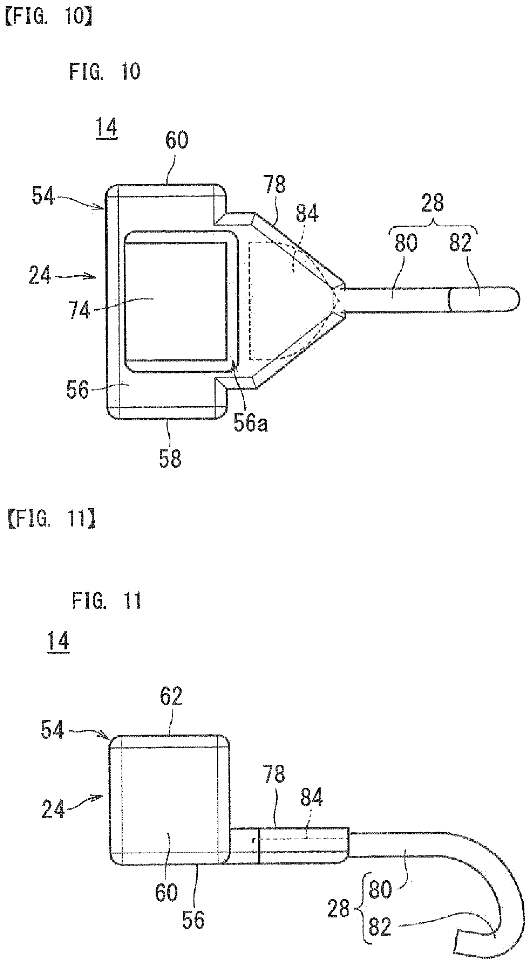

Next, the second straightening member 14 is described in detail with reference to, for example, FIG. 8, FIG. 9, FIG. 10, FIG. 11, FIG. 12, and FIG. 13.

The fastening locking part 24 of the second straightening member 14 includes, for example, a housing 54 having a rectangular cylindrical cross section, as depicted in FIG. 8 to FIG. 13. The housing 54 has an insertion hole 22 penetrating to the width direction of the housing 54 and having, for example, a rectangular cross section. In this case, the housing 54 includes a lower wall surface part 56 having a through hole 56a in, for example, a rectangular shape. At one end and the other end of the lower wall surface part 56 in the length direction, a right wall surface part 58 and a left wall surface part 60 each having, for example, a square shape, are respectively provided to stand upright. Furthermore, between an upper end of the right wall surface part 58 and an upper end of the left wall surface part 60, an upper wall surface part 62 having, for example, a rectangular shape, is provided to spread. Also, the upper wall surface part 62 has, on one end side in its width direction, a notched part 62a having a C shape in a planar view, as depicted in FIG. 8 and FIG. 9. The notched part 62a is provided so that the lock nail part 26, which will be described further below, is partially exposed.

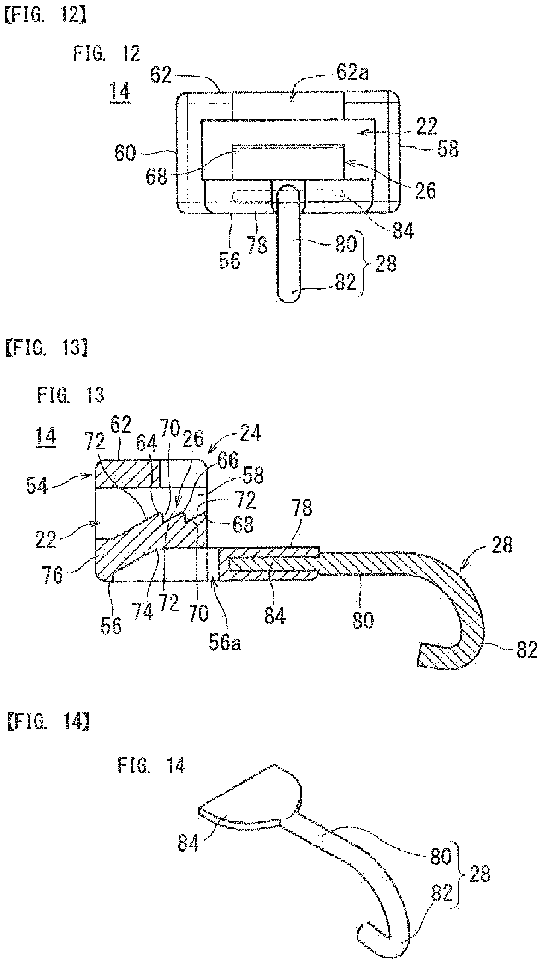

In this fastening locking part 24, as depicted in FIG. 8 and FIG. 13, the lock nail part 26 is disposed inside the fastening locking part 24. The lock nail part 26 is provided to extend from one end edge part to a portion near the other end edge part of the through hole 56a of the lower wall surface part 56 in the width direction. On one main surface (upper surface) of the lock nail part 26, for example, three nail pieces 64, 66, and 68 are disposed. The three nail pieces 64, 66, and 68 each include an upright engaging surface 70 to be engaged with the upright surface 42 of the sawtooth part 40 provided on the belt part 18 of the first straightening member 12 and a tilted engaging surface 72 to be engaged with the tilted surface 44 of the sawtooth part 40.

When an angle at which the upright engaging surface 70 and the tilted engaging surface 72 of the lock nail part 26 cross is taken as θ_{L}, θ_{L} is set so as to be in a range of 45°<θ_{L}<60°. Also, the above-described angle θ_{B} at which the upright surface 42 and the tilted surface 44 of the sawtooth part 40 cross and the angle θ_{L} at which the upright engaging surface 70 and the tilted engaging surface 72 of the lock nail part 26 cross are set to be equal.

Also, three nail pieces 68, 70, and 72 of this lock nail part 26 are disposed so as to be in a movable state via a cantilever-like movable piece 74 with one end edge part of the through hole 56a of the lower wall surface part 56 in the width direction being taken as a fixed end, as depicted in FIG. 13. The movable piece 74 includes, for example, a hinge part 76 disposed at one end edge part of the lower wall surface part 56 in the width direction. The movable piece 74 is supported by the hinge part 76 so as to be able to freely rock to a direction orthogonal to the inserting direction of the belt part 18 of the first straightening member 12.

Note in this embodiment of the invention that while the lock nail part 26 is formed of, for example, three nail pieces 64, 66, and 68, the number is not necessarily required to be three, and may be, for example, one or two, or four or more.

To the fastening locking part 24 of this second straightening member 14, as depicted in FIG. 8, FIG. 10, and FIG. 11, a second support part 78 for supporting the second latching part 28 is disposed. This second support part 78 is formed in, for example, a flat plate shape and in a substantially triangular shape in a planar view, and is connected to the other end side of the lower wall surface part 56 in the width direction. The second support part 78 is provided to extend outward from the other end edge part of the through hole 56a of the lower wall surface part 56 in the width direction. The second support part 78 is disposed in parallel with the upper wall surface part 62.

The second latching part 28 is formed in a manner similar to that of the first latching part 20. That is, the second latching part 28 includes a stem part 80, as depicted in FIG. 8 to FIG. 11 and FIG. 13. The stem part 80 has one end side in the axial direction supported by the second support part 78 and has a hook part 82 formed on the other end side in the axial direction.

The fastening locking part 24, the lock nail part 26, and the second support part 78 of the second straightening member 14 are integrally formed of a flexible material, for example, a plastic material.

Also, the stem part 80 and the hook part 82 of the second latching part 28 are integrally formed of an elastic material, for example, a metal material. Furthermore, the second latching part 28 includes a second insert part 84 as depicted in FIG. 14, and the second insert part 84 is insert-molded by, for example, injection molding, so as to be supported by the second support part 78. In this case, a root part side of the stem part 80 of the second latching part 28, that is, the second insert part 84 to be inserted into the second support part 78, is formed in, for example, a semi-circular flat shape in a planar view as a rotation-stop and slip-stop structure, as depicted in FIG. 13.

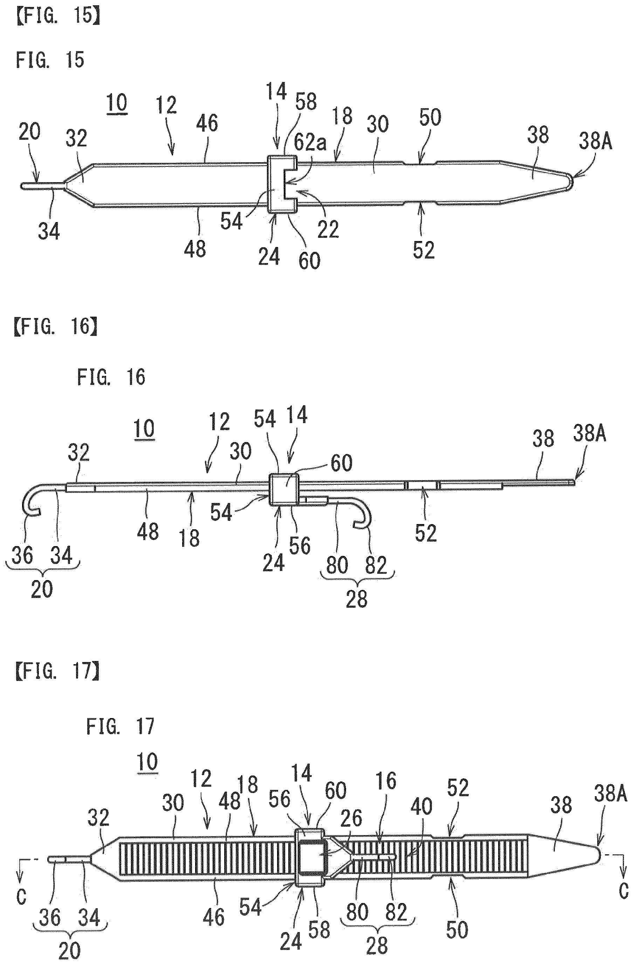

Next, the structure of fastening the belt part 18 by the first straightening member 12 and the second straightening member 14 is described in detail below with reference to FIG. 15, FIG. 16, FIG. 17, FIG. 18, FIG. 19, and FIG. 20.

The structure of fastening the belt part 18 by the first straightening member 12 and the second straightening member 14 is such that the belt part 18 of the first straightening member 12 is inserted into the insertion hole 22 of the fastening locking part 24 of the second straightening member 14 and the length of the belt part 18 to be pulled out from the insertion hole 22 is adjusted.

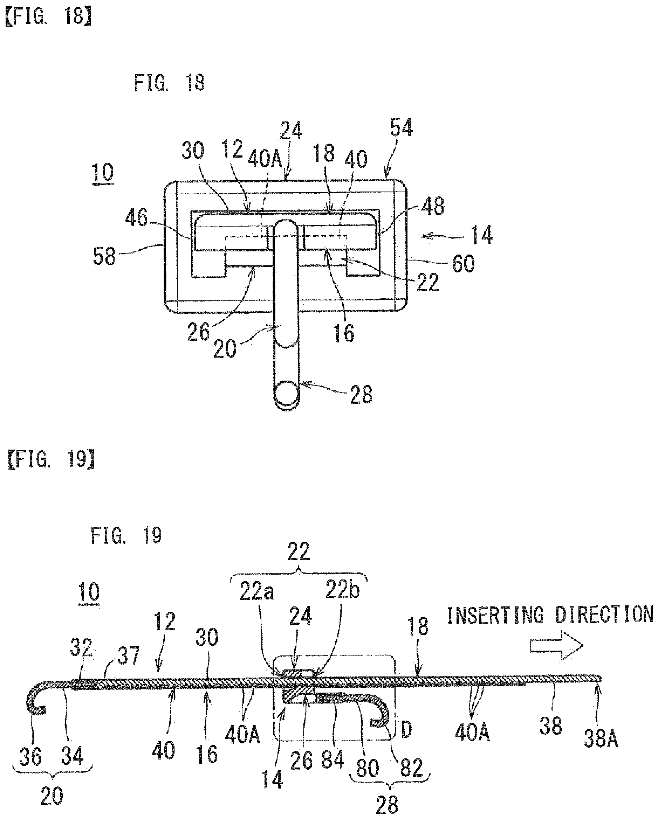

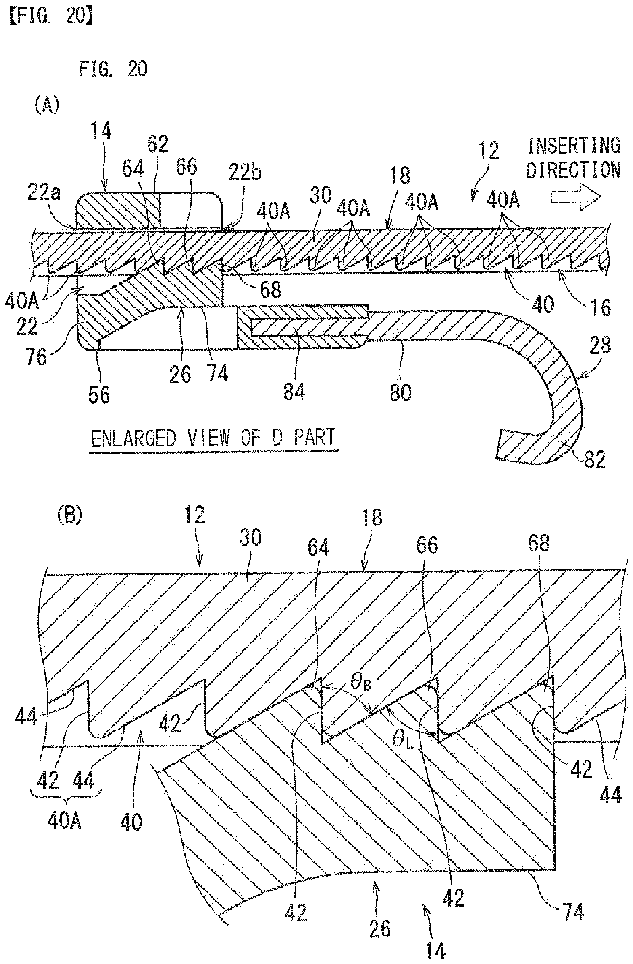

That is, when the belt part 18 is inserted into an entrance 22a of the insertion hole 22 of the fastening locking part 24 and is pulled out from an exit 22b of the insertion hole 22, in particular, for example, as depicted in FIG. 19 and FIG. 20, the nail pieces 64, 66, and 68 of the lock nail part 26 are each engaged, by using its elastic force, mutually with any sawtooth piece 40A among the plurality of sawtooth pieces 40A of the sawtooth part 40 of the belt part 18 to fix the belt part 18. With any sawtooth piece 40A among the plurality of sawtooth pieces 40A engaged with each of the nail pieces 64, 66, and 68 of the lock nail part 26, the fixing position of the belt part 18 in the insertion hole 22 of the fastening locking part 24 can be adjusted to set a fastening length of the belt part 18.

According to the device 10 in accordance with the embodiment described above, the first latching part 20 of the first straightening member 12 is latched at one end edge of a deformed nail surface N in the width direction, and the second latching part 28 of the second straightening member 14 is latched at the other end edge of the nail surface N in the width direction. Furthermore, the free end 38A side of the belt part 18 is inserted into the insertion hole 22 of the lock nail part 26 of the fastening locking part 24, and the free end 38A side of the belt part 18 is pulled, thereby allowing the belt part 18 to be fastened to the nail surface N.

In this case, the one end edge and the other end edge sides of the nail surface N in the width direction are pulled by the first latching part 20 and the second latching part 28, respectively, to a direction opposite to the ingrowing direction of the deformed nail surface N, for example, "ingrown nail". This tensile force can be increased as the length of the belt part 18 to be pulled out from the insertion hole 22 of the fastening locking part 24 is longer, that is, a length between one end part of the belt part 18 in the length direction and a portion of the belt part 18 locked by the lock nail part 26 is shorter. By contrast, this tensile force can be decreased as the length of the belt part 18 to be pulled out from the insertion hole 22 of the fastening locking part 24 is shorter, that is, the length between one end part of the belt part 18 in the length direction and the portion of the belt part 18 locked by the lock nail part 26 is longer. Therefore, this tensile force can be adjusted as appropriate in accordance with the size and shape of the deformed nail surface N, the degree of a curve of the ingrown nail, and so forth.

And, the tensile force adjusted by this adjustment is retained by the lock function of the lock nail part 26 inhibiting the belt part 18 from returning to the direction opposite to the inserting direction of the belt part 18. Thus, the tensile force to the direction opposite to the ingrowing direction of the ingrown nail continuously acts at both end edges of the ingrown nail in the width direction, and a decrease in this tensile force is prevented. Therefore, according to this device 10, deformity of the nail such as ingrown nail can be straightened.

Also as described above, in this device 10, when the belt part 18 is fastened by the fastening locking part 24, the belt part 18 makes contact with the surface of the nail surface N and pressurizes the nail surface N downward. Thus, in particular, with the belt part 18 as a center, the first latching part 20, the fastening locking part 24, and the second latching part 28 are each stably attached to the surface of the nail surface N without being shifted or moved.

Since this device 10 has the structure and operations described above, compared with the conventional technology described in, for example, Patent Literature 1, the work of twisting the coupling members by using the dedicated tool or the work of twining the coupling members when not using the dedicated tool is not required.

Thus, in this device 10, burdensome efforts to attach this device 10 to the nail surface N are not required, and the attachment time is also short. Also in the conventional technology, when the work of twisting or twining the coupling members is performed, a boundary portion between a force introduction member and a coupling member may be broken by bending. By contrast, in this device 10, these twisting work and twining work are not required, and thus inconveniences such as breakage of the device itself by bending are eliminated.

That is, according to this device 10, the device can be easily attached in an effort-saving manner without damage on the device at the time of attachment, and an appropriate straightening force can be caused to act over a long period. Also, the straightening force can be caused to act as intended by the user.

Also, in this device 10, the first support part 32 can stably support the first latching part 20 to the nail surface N and, furthermore, the lead part 38 can easily guide the belt part 18 to the second straightening member 14. Also, since the sawtooth part 40 is disposed so as to be opposed to the nail surface N, when this device 10 is attached to the nail surface N, the sawtooth part 40 makes contact with the deformed nail surface N. Thus, the sawtooth part 40 can prevent a positional shift due to slipping of the belt part 18 on the nail surface N.

Also, in this device 10, the upright surface 42 of the sawtooth part 40 and the upright engaging surface 70 of the lock nail part 26 are engaged together, and the tilted surface 44 of the sawtooth part 40 and the tilted engaging surface 72 of the lock nail part 26 are engaged together. Thus, when the belt part 18 is inserted into the insertion hole 22 of the fastening locking part 24, the tilted engaging surface 72 of the lock nail part 26 is pressurized by the tilted surface 44 of the sawtooth part 40 to cause the belt part 18 to move to the inserting direction. Also, when the belt part 18 is about to return to the direction opposite to the inserting direction of the belt part 18, the upright surface 42 of the sawtooth part 40 and the upright engaging surface 70 of the lock nail26 part are engaged, and a surface contact is made with that engaging area. Thus, the lock function against the return force applied to the belt part 18 is firmly exerted.

Also, in this device 10, one end side of the lock nail part 26 serves as a fixed end supported to the inside of the fastening locking part 24, and the other end of the lock nail part 26 serves as a free end to allow movement. When the belt part 18 is inserted into the insertion hole 22 of the fastening locking part 24, the tilted engaging surface 72 of the lock nail part 26 is pressurized as sliding over the tilted surface 44 of the sawtooth part 40 to be warped to the inserting direction of the belt part 18. Thus, the belt part 18 can move through the insertion hole 22 to the inserting direction. Also, when the belt part 18 is tried to return to the direction opposite to the inserting direction of the belt part 18, the upright surface 42 of the sawtooth part 40 and the upright engaging surface 70 of the lock nail part 26 are engaged, and a resistance force acts against the return force of the belt part 18, thereby locking the return of the belt part 18.

Furthermore, in this device 10, when an angle at which the upright surface 42 and the tilted surface 44 of the sawtooth part 40 cross is taken as θ_{B}, θ_{B} is set so that 45°<θ_{B}<60°; when an angle at which the upright engaging surface 70 and the tilted engaging surface 72 of the lock nail part 26 cross is taken as θ_{L}, θ_{L} is set so that 45°<θ_{L}<60°; and, furthermore, it is set so that θ_{B}=θ_{L}. Thus, when the belt part 18 is inserted into the insertion hole 22 of the fastening locking part 24, a tilted angle between the tilted surface 44 of the sawtooth part 40 and the tilted engaging surface 72 of the lock nail part 26 is a tilted angle suitable for the belt part 18 to move to the inserting direction. Thus, the belt part 18 can easily move to the fastening direction via the insertion hole 22 of the fastening locking part 24 and, in turn, fastening of the belt part 18 by the fastening locking part 24 onto the nail surface N is facilitated.

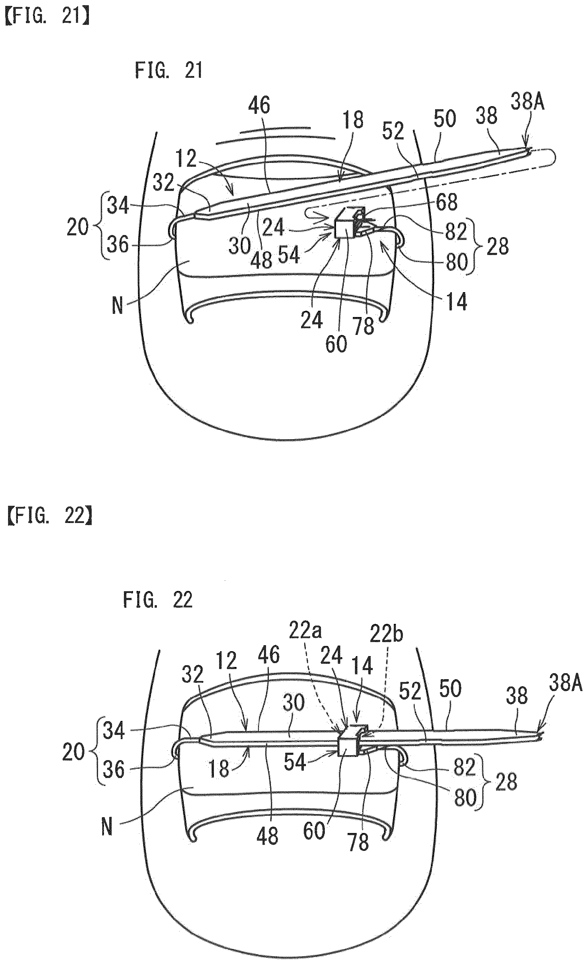

Next, one example of a method of using the nail deformity correction device 10 according to the embodiment of this invention is described in detail below with reference to FIG. 21 to FIG. 26.

FIG. 21 is a perspective view depicting one example of a process of attaching the nail deformity correction device depicted in FIG. 1 and FIG. 2 to a deformed nail, and is a perspective view depicting a process of, for example, latching the first latching part of the first straightening member at one end edge of a nail in a width direction and latching the second latching part of the second straightening member at the other end edge of the nail in the width direction.

First, the first straightening member 12 and the second straightening member 14 are prepared, and a substantial entirety of a deformed nail surface N attached to this device is sanitized in advance as appropriate.

Next, the first latching part 20 of the first straightening member 12 is nipped by, for example, a needle holder (not depicted) and, as depicted in FIG. 21, the hook part 36 of this first latching part 20 is latched at a location of substantially one third of a nail matrix (nail root side) of a nail groove on one end edge side of the nail surface N in the width direction. The reason for this is that if the latching position of the hook part 36 of the first latching part 20 is too close to the nail matrix (nail root side), there is a fear of affecting nail formation.

Also, on the other end edge side of the nail surface N in the width direction, the second latching part 28 of the second straightening member 14 is latched at a location of substantially one third of the nail matrix (nail root side) of the nail groove on the other end edge side of the nail surface N in the width direction, as depicted in FIG. 21.

Next, the belt part 18 of the first straightening member 12 on its free end 38A side is inserted into the insertion hole 22 of the fastening locking part 24 of the second straightening member 14. In this case, the right-side constricted part 50 and the left-side constricted part 52 of the belt part 18 are nipped by hand fingers or a needle holder (not depicted) and, as depicted in FIG. 22, the belt part 18 is led by the lead part 38 to be inserted from the entrance 22a of the insertion hole 22 of the fastening locking part 24 and be pulled out from the exit 22b of the insertion hole 22.

In this case, the pulled length of the belt part 18 pulled out from the exit 22b of the insertion hole 22 is adjusted, and the sawtooth part 40 of the belt part 18 is locked by the lock nail part 26 of the fastening locking part 24 at a position where a tension applies between the first latching part 20 and the second latching part 28 to cause the belt part 18 to become stretched taut. That is, the fixing position where the belt part 18 is locked is adjusted, and the fastening length of the belt part 18 with respect to the nail surface N is determined.

Here, as for each of the first latching part 20 and the second latching part 28, one end edge part and the other end edge part of the nail surface N in the width direction are pulled up by any tensile force desired by the user. That is, the one end edge part and the other end edge part of the nail surface N in the width direction are pulled up by the hook part 36 of the first latching part 20 and the hook part 82 of the second latching part 28 to the direction (upward direction) opposite to, for example, the ingrowing direction of the ingrown nail.

The above-described tensile force can be increased as the length of the belt part 18 pulled out from the exit 22b of the insertion hole 22 of the fastening locking part 24 is made longer, that is, as the length between one end of the belt part 18 in the length direction and the portion of the belt part 18 locked by the lock nail part 26 is shorter, for example, as depicted in FIG. 19, FIG. 20 and FIG. 21, and FIG. 22. By contrast, this tensile force can be decreased as the length of the belt part 18 pulled out from the exit 22b of the insertion hole 22 of the fastening locking part 24 is made shorter, that is, as the length between one end of the belt part 18 in the length direction and the portion of the belt part 18 locked by the lock nail part 26 is longer. Therefore, this tensile force can be adjusted in accordance with the size and shape of the nail surface N of the deformed ingrown nail, the degree of the curve of the ingrown nail, and so forth.

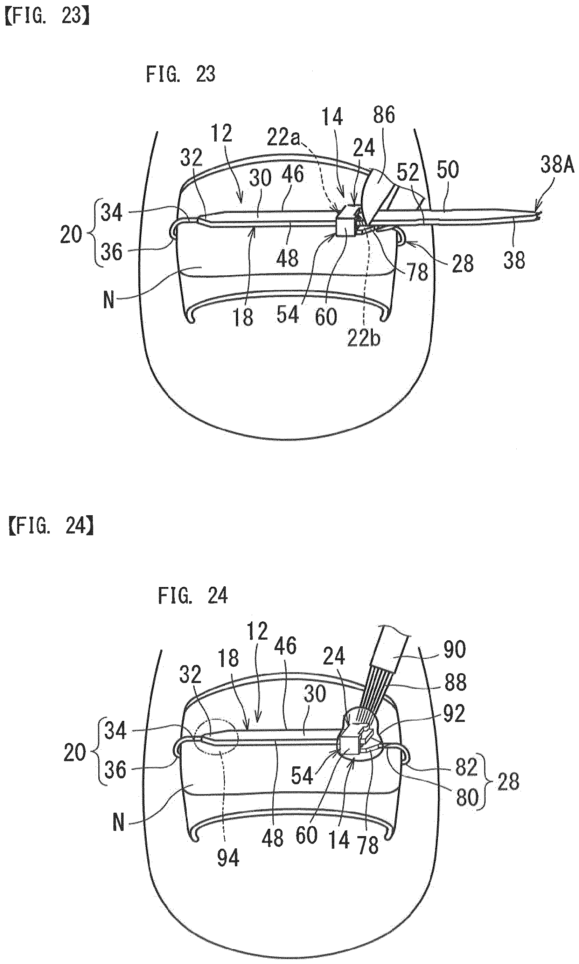

After the fastening length with respect to the nail surface N of the belt part 18 is determined in this manner, the remaining portion of the belt part 18 pulled out from the exit 22b of the insertion hole 22 of the fastening locking part 24 is next cut out by cutting-out means 86 such as a cutter at a portion near the exit 22b of the insertion hole 22, as depicted in FIG. 23.

Furthermore, in this embodiment, as depicted in FIG. 24, a coating material 88 such as, for example, a paste-like ultraviolet-curable resin material is applied from a container 90 such as a tube or is applied with an applying tool such as a brush (not depicted) so as to cover, for example, the fastening locking part 24, the second support part 78, a boundary part between the second support part 78 and the second latching part 78, and its surroundings of the second straightening member 14, thereby forming an application part 92. Also, similarly, an application part 94 can be formed as appropriate at a boundary part between the first latching part 20 and the first support part 32 of the first straightening member 12.

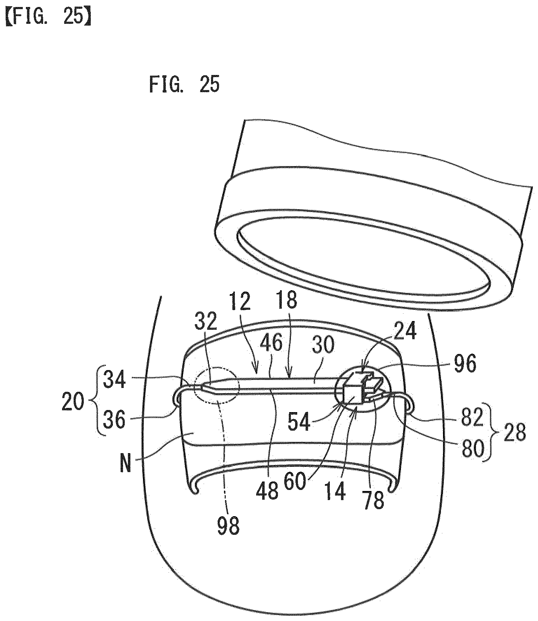

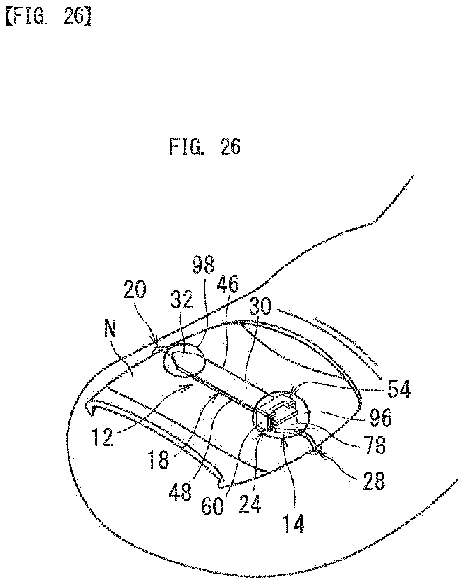

Then, the entirety of each of the application parts 92 and 94 is irradiated with ultraviolet rays by, for example, an ultraviolet LED (ultraviolet light-emitting diode), for a predetermined time, for example, on the order of twenty seconds, as depicted in FIG. 25. This cures the application parts 92 and 94, and coated parts 96 and 98 are formed, respectively, as depicted in FIG. 26. The coated part 96 protects the fastening locking part 24, the second support part 78, the boundary part between the second support part 78 and the second latching part 78, and its surroundings. Also, the coated part 98 protects the boundary part between the first latching part 20 and the first support part 32 of the first straightening member 12 and its surrounding. Furthermore, the coated parts 96 and 98 have a function of stably maintaining the tensile force of pulling the one end edge part and the other end edge part of the nail surface N in the width direction upward.

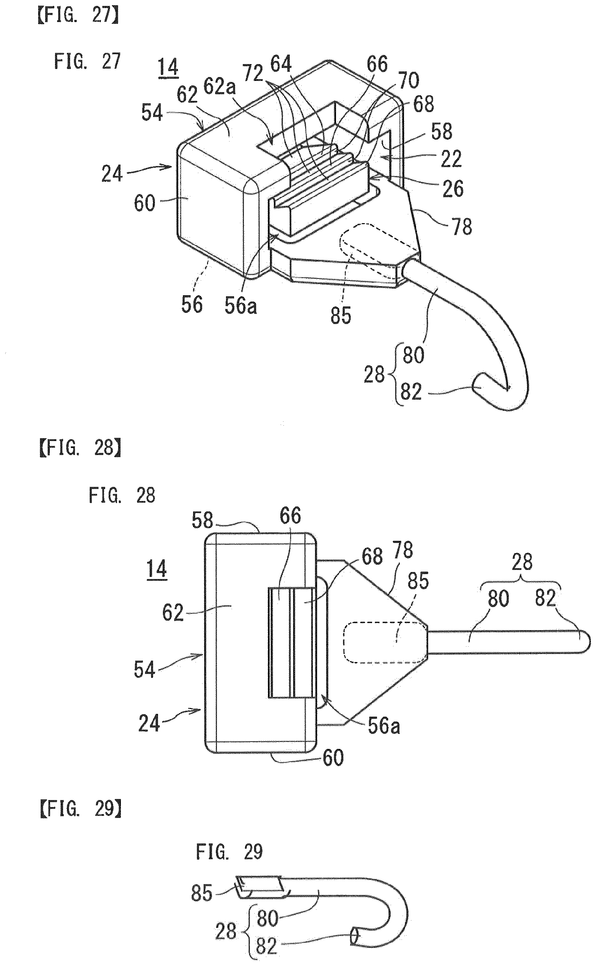

FIG. 27 is a perspective view depicting a modification example of the second straightening member that can be applied to the nail deformity correction device according to the present invention. FIG. 28 is a plan view of the second straightening member depicted in FIG. 27. FIG. 29 is a perspective view depicting a second insert part of a second latching part depicted in FIG. 27 and FIG. 28.

In this modification example, compared with the device 10 according to the embodiment described above, in particular, the shape of a second insert part 85 of the second latching part 28 is different. That is, while the second insert part 84 of the second latching part 28 is formed in, for example, a semi-circular flat shape in a planar view in the above-described embodiment, the second insert part 85 of the second latching part 88 in the second straightening member 14 according to the modification example depicted in FIG. 27 and FIG. 28 is formed in, for example, a rectangular shape in a planar view.

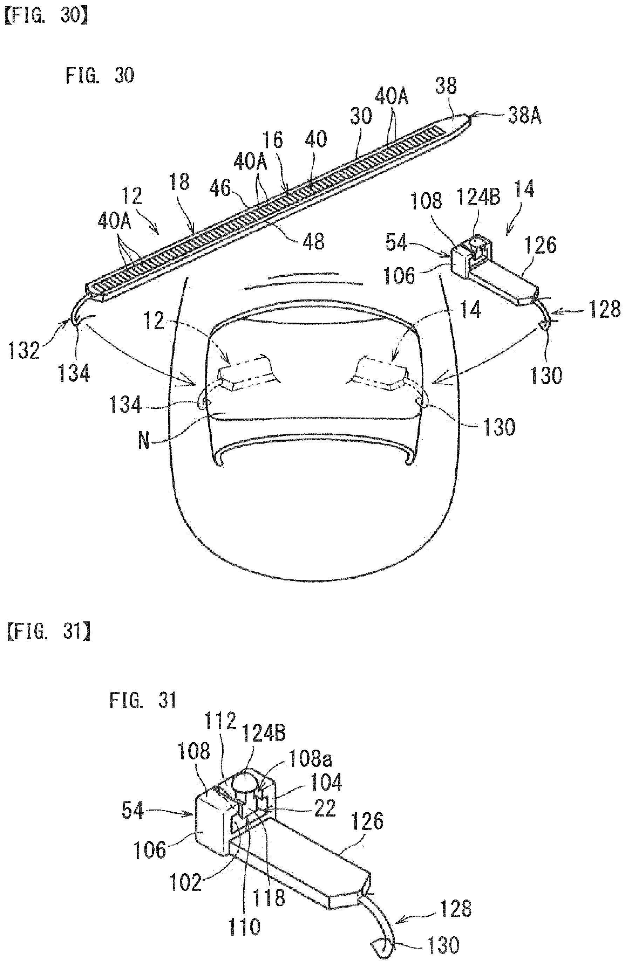

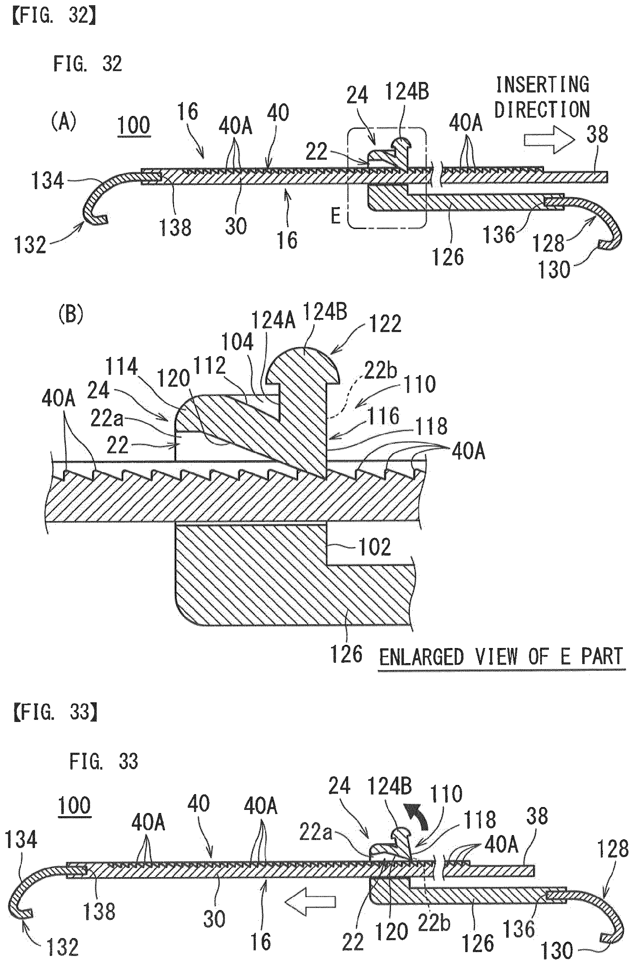

FIG. 30 depicts another example of the embodiment of the nail deformity correction device according to the present invention, and is a perspective view depicting one example of a process of attaching a first straightening member and a second straightening member of the nail deformity correction device to a deformed nail. FIG. 31 is perspective view depicting one example of the second straightening member to be applied to the nail deformity correction device depicted in FIG. 30. FIG. 32(A) is a sectional view depicting a state when the first straightening member is inserted into the second straightening member depicted in FIG. 30 and FIG. 32(B) is an enlarged view of an E part of FIG. 32(A). Furthermore, FIG. 33 is a sectional view of an operation state of releasing an engaged state between a sawtooth part of the first straightening member and a lock nail part of the second straightening member from the state depicted in FIG. 32.

The device 100 according to this embodiment is different, in particular, in the structure of the fastening locking part 24 of the second straightening member 14, compared with the device 10 described above. In the following, different points compared with the device 10 are mainly described. Also, among reference signs written in the drawings of FIG. 30 to FIG. 33, members with same reference signs used in the description of the device 10 according to the embodiments described above have similar structure, operation, and effect, and therefore detailed description of these members are omitted.

That is, in the second straightening member 14 of the device 100 according to this embodiment, the fastening locking part 24 of the second straightening member 14 includes, as depicted in FIG. 30 and FIG. 31, the housing 54 having, for example, a rectangular cylindrical cross section. The housing 54 has the insertion hole 22 penetrating to the width direction of the housing 54 and having, for example, a rectangular cross section. The housing 54 includes a lower wall surface part 102 having, for example, a rectangular shape. At one end and the other end of the lower wall surface part 102 in the longitudinal direction, a right wall surface part 104 and a left wall surface part 106 each having, for example, a rectangular shape, are respectively provided to stand upright. Furthermore, between an upper end of the right wall surface part 104 and an upper end of the left wall surface part 106, an upper wall surface part 108 having, for example, a rectangular shape, is provided to spread. The upper wall surface part 108 has a notched part 108a having a U shape in a planar view as depicted in FIG. 31. The notched part 108a is provided from one end side to the other end side of the upper wall surface part 108 in the width direction and has a predetermined length at an intermediate part of the upper wall surface part 108 in the longitudinal direction. This notched part 108a is provided so that a lock nail part 110, which will be described further below, is partially exposed.

In the fastening locking part 24, as depicted in FIG. 31 and FIG. 32, the lock nail part 110 is disposed inside the fastening locking part 24. The lock nail part 110 includes a surrounding portion 112 surrounded by the notched part 108a of the upper wall surface part 108. The surrounding portion 112 is connected to one end of the upper wall surface 108 in the width direction. The surrounding portion 112 has the one end in the width direction as a fixed end and the other end in the width direction as a free end. Thus, the free end side of the surrounding portion 112 is formed so as to freely rock in a vertical direction. In this case, the fixed end side of the surrounding portion 112 includes a hinge part 114. By the hinge part 114, the free end side can freely lock.

In the lock nail part 110, on a lower surface of the surrounding portion 112 opposed to the lower wall surface part 102, for example, one nail piece 116 is disposed. The nail piece 116 each includes an upright engaging surface 118 to be engaged with the upright surface 42 of the sawtooth part 40 provided to the belt part 18 of the first straightening member 12 and a tilted engaging surface 120 to be engaged with the tilted surface 44 of the sawtooth part 40. In the device 100 of this embodiment, compared with the device 10 of the embodiment described above, the sawtooth part 40 included in the belt part 18 of the first straightening member 12 is formed on the other main surface (upper surface) of the belt part main body 30. In this case, when an angle at which the upright engaging surface 118 and the tilted engaging surface 120 of the nail piece 116 cross is taken as θ_{L}, as with the device 10 of the embodiment described above, θ_{L} is set so as to be in a range of 45°<θ_{L}<60°. Also, the above-described angle θ_{B} at which the upright surface 42 and the tilted surface 44 of the sawtooth part 40 cross and the angle θ_{L} at which the upright engaging surface 118 and the tilted engaging surface 120 of the nail piece 116 cross are set to be equal.

The nail piece 116 of this lock nail part 110 has a structure of the above-described surrounding portion 112 as depicted in FIG. 32(B) and is thus formed in a cantilever manner such that, with the one end of the upper wall surface part 108 in the width direction as a fixed end, its free end side is in a movable state. That is, the lock nail part 10 is supported by the hinge part 114 so as to freely rock to a direction orthogonal to the inserting direction of the belt part 18 of the first straightening member 12 inserted into the insertion hole 22 of the fastening locking part 24.

Furthermore, the fastening locking part 24 further includes an activation piece 122 connected to the lock nail part 110 and exposed to outside the fastening locking part 24. The activation piece 122 includes a neck part 124A having a rectangular cross section, as depicted in FIG. 31 and FIG. 32(B). A lower end part of the neck part 124A is connected to the nail piece 116 of the lock nail part 110, and a side end surface of the neck part 124A is connected to the end surface of the upright engaging surface of the nail piece 116 so as to be flush therewith. Also, at an upper end of the neck part 124A, a knob part 124B having, for example, a spherical crown shape, is connected. In this case, the lock nail part 110 and the activation piece 122 are integrally formed.

In this embodiment, as depicted in FIG. 31 and FIG. 32(A), a second support part 126 is formed in a flat plate shape having, for example, a substantially narrowly-elongated rectangular shape in a planar view, and is connected to the other end side of the lower wall surface part 102 in the width direction. The second support part 126 is disposed in parallel with the upper wall surface part 108. Also, a second latching part 128 does not have the stem part 80 compared with the second latching part 28 of the device 10 described above, and is formed of only a hook part 130. Furthermore, the second latching part 128 includes a second insert part 136 having, for example, a stem shape, compared with the second insert part 84 of the device 10 described above, and the second insert part 136 is insert-molded by, for example, injection molding, so as to be supported by the second support part 126. In this case, in a root part side of the hook part 130 of the second latching part 128, that is, in the second insert part 136 to be inserted into the second support part 126, for example, knurls (not depicted) are formed as a rotation-stop and slip-stop structure on the periphery of the insert part 136, as depicted in FIG. 32(A).

Note that a first latching part 132 is formed of only the hook part 134 identical in shape and size to the hook part 130 of the second latching part 128. Also, the first latching part 132 is not provided with a first support part dish, compared with the second latching part 28 of the device 10 described above, and the first latching part 132 is disposed at one end of the belt part 18 in the length direction. In this case, the first latching part 132 includes a first insert part 138 having a stem shape, and the first insert part 138 is insert-molded by, for example, injection molding, so as to be supported by one end of the belt part 18 in the length direction.

In this embodiment, the fastening locking part 24, the lock nail part 110, and the second support part 126 are integrally formed of a flexible material, for example, a plastic material. Also, the hook part 130 and the insert part 136 of the second latching part 128 are integrally formed of an elastic material, for example, a metal material. Similarly, the hook part 134 and the insert part 138 of the first latching part 132 are integrally formed of an elastic material, for example, a metal material.

According to the device 100 in accordance with the embodiment depicted in FIG. 30 to FIG. 33, the activation piece 122 can pull up the lock nail part 110 to a direction orthogonal to the inserting direction of the belt part 18 and isolating the engagement between the sawtooth piece 40A of the sawtooth part 40 and the nail piece 116 of the lock nail part 110. In this case, a knob part 124B of the activation piece 122 is nipped or the like by hand fingers or a needle holder (not depicted), thereby allowing the lock nail part 110 to be pulled upward, as depicted in FIG. 33. Thus, the engaged state between the upright surface 42 of the sawtooth piece 40A of the sawtooth part 40 and the upright engaging surface 118 of the lock nail part 110 and the engaged state between the tilted surface 44 of the sawtooth piece 40A of the sawtooth part 40 and the tilted engaging surface 120 of the lock nail part 110 can be released.

Therefore, in the device 100 in accordance with this embodiment, the belt part 18 can be moved to a direction opposite to the inserting direction of the belt part 18. Thus, even after the device 100 is attached to the nail surface N, the belt part 18 is moved as appropriate from the insertion hole 22 of the fastening locking part 24 to the direction opposite to the inserting direction to once loosen fastening of the belt part 18 and adjust the strength of fastening, thereby allowing the straightening force applied to the nail surface N to be freely and easily changed. Furthermore, according to this device 100, the belt part 18 can be pulled out from the insertion hole 22 of the fastening locking part 24 for reuse of the first straightening member 12 once used.

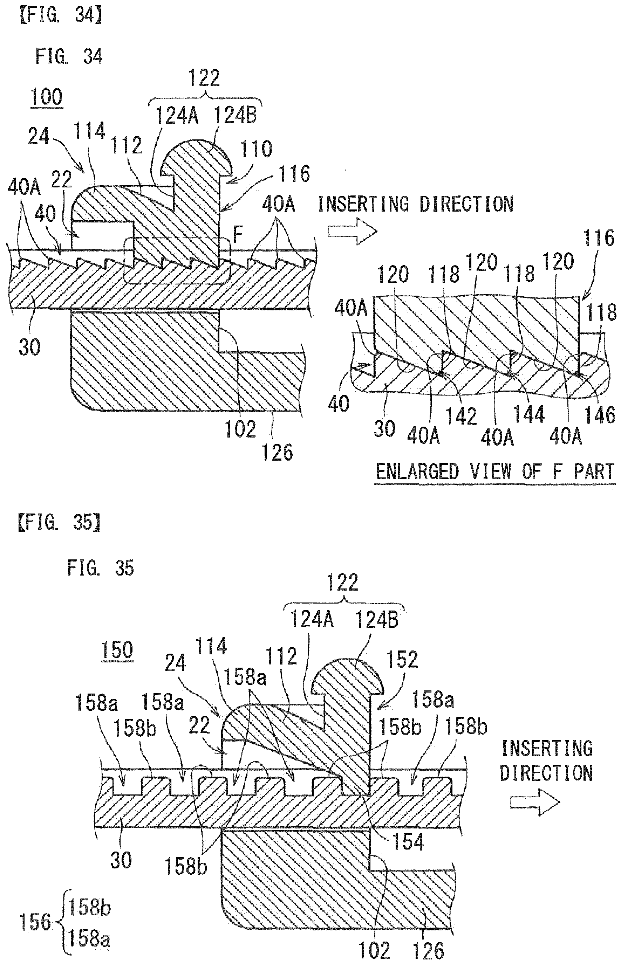

FIG. 34 depicts a modification example of the second straightening member depicted in FIG. 30 and FIG. 31 and is an enlarged sectional view of main parts depicting a state in which the first straightening member is inserted into the second straightening member. In this modification example, compared with the second straightening member 14 according to the device 100 of the above-described embodiment, in particular, the number of nail pieces of the lock nail part 110 is different.

That is, in the lock nail part 110 according to the device 100 depicted in FIG. 30 to FIG. 33, for example, only one nail piece 116 is provided. In the second straightening member 14 of this modification example, the lock nail part 110 includes, for example, three nail pieces 140a, 140b, and 140c. Also, the number of nail pieces of this lock nail part 110 may be, for example, two or four or more.

FIG. 35 depicts still another example of the embodiment of the nail deformity correction device according to the present invention and is a sectional view depicting a state in which the first straightening member of the nail deformity correction device is inserted into the second straightening member.

A device 150 according to this embodiment is different, in particular, in the structure of a fastening locking part of the second straightening member, compared with the device 10 and the device 100 according to the embodiments described above. In the following, different points compared with the device 10 and the device 100 are mainly described. Also, among reference signs written in the drawing of FIG. 35, members with same reference signs used in the description of the device 10 and the device 100 according to the embodiments described above have similar structure, operation, and effect, and therefore detailed description of these members are omitted.

That is, in the device 150 according to this embodiment, as depicted in FIG. 35, it is characterized in that, in particular, the fastening locking part 24 of the second straightening member 14 includes a lock nail part 152 formed of a single nail piece 154 and the first straightening member 12 includes concave/convex part 156 to be locked to the nail piece 154 of the lock nail part 152.

The lock nail part 152 of the fastening locking part 24 according to this device 150 includes the nail piece 154 formed of, for example, a stem-shaped part. This nail piece 154 can be formed to have its sectional shape being a circular shape, rectangular shape, square shape, oval shape, or any other shape.

On the other hand, on the belt part 18 of the first straightening member 12, a serration part 156 to be locked to the nail piece 154 of the lock nail part 152 is disposed. In this case, the serration part 156 is characterized to include, as depicted in FIG. 35, a plurality of concave parts 158a and convex parts 158b consecutively provided.

According to the device 150 in accordance with the embodiment depicted in FIG. 35, the activation piece 122 can pull up the lock nail part 152 to a direction orthogonal to the inserting direction of the belt part 18 and releasing a locked state between the concave part 156a and the convex part 156b of the serration part 156 and the nail pieces 154 of the lock nail part 152. That is, the knob part 124B of the activation piece 122 is nipped or the like by, for example, hand fingers or a needle holder (not depicted) to pull the lock nail part 152 upward. Thus, the nail piece 154 of the lock nail part 152 can be isolated from the concave parts 156a of the serration part 156.

Therefore, in the device 150 according to this embodiment, the belt part 18 can be moved to a direction opposite to the inserting direction of the belt part 18. Thus, even after the device 150 is attached to the nail surface N, the belt part 18 is moved as appropriate from the insertion hole 22 of the fastening locking part 24 to the direction opposite to the inserting direction to once loosen fastening of the belt part 18 and adjust the strength of fastening, thereby allowing the straightening force applied to the nail surface N to be freely and easily changed. Furthermore, according to this device 150, the belt part 18 can be pulled out from the insertion hole 22 of the fastening locking part 24 for reuse of the first straightening member 12 once used.

### Reference Signs List

- 10: nail deformity correction device
- 12: first straightening member
- 14: second straightening member
- 16: serration part
- 18: belt part
- 20: first latching part
- 22: insertion hole
- 24: fastening locking part
- 26: lock nail part
- 28: second latching part
- 30: belt part main body
- 32: first support part
- 34: stem part
- 36: hook part
- 37: first insert part
- 38: lead part
- 38A: free end
- 40: sawtooth part
- 40A: sawtooth piece
- 42: upright surface
- 44: tilted surface
- 46: right side wall part
- 48: left side wall part
- 50: right-side constricted part
- 52: left-side constricted part
- 54: housing
- 56: lower wall surface part
- 58: right side wall surface part
- 60: left side wall surface part
- 62: upper wall surface part
- 62a: notched part
- 64, 66, 68: nail piece
- 70: upright engaging surface
- 72: tilted engaging surface
- 74: movable piece
- 76: hinge part
- 78: second support part
- 80: stem part
- 82: hook part
- 84, 85: second insert part
- 86: cutting-out means
- 88: coating material
- 90: container
- 92, 94: application part
- 96, 98: coated part
- 100: nail deformity correction device
- 102: lower wall surface part
- 104: right side wall surface part
- 106: left side wall surface part
- 108: upper wall surface part
- 110: lock nail part
- 112: surrounding portion
- 114: hinge part
- 116: nail piece
- 118: upright engaging surface
- 120: tilted engaging surface
- 122: activation piece
- 124A: neck part
- 124B: knob part
- 126: second support part
- 128: second latching part
- 130, 134: hook part
- 132: first latching part
- 136: first insert part
- 138: second insert part
- 142, 144, 146: nail piece
- 150: nail deformity correction device
- 152: lock nail part
- 154: nail piece
- 156: concave/convex part
- 158a: concave part
- 158b: convex part
- N: nail surface of a deformed nail

## Claims

1. A nail deformity correction device comprising a first straightening member and a second straightening member which correct deformity of a finger nail of a foot in cooperation with each other,
the first straightening member including
a belt part having a serration part, and
a first latching part disposed at one end of the belt part in a length direction to be latched at one end edge of the deformed nail in a width direction, and
the second straightening member including
a fastening locking part having an insertion hole into which the belt part is inserted to fasten and lock the belt part to the deformed nail,
a lock nail part disposed inside the fastening locking part to be locked to the serration part of the belt part inserted into the insertion hole to inhibit the belt part from returning to a direction opposite to an inserting direction of the belt part, and
a second latching part formed to extend from the fastening locking part to the inserting direction of the belt part to be latched at another end edge of the deformed nail in the width direction.

2. The nail deformity correction device according to claim 1, wherein
the belt part further includes
a belt part main body,
a first support part disposed at one end of the belt part main body in a length direction to support the first latching part, and
a lead part disposed at another end of the belt part main body in the length direction and having a free end at a tip, to guide the belt part to the second straightening member, and
the serration part is disposed between the first support part and the lead part and at an intermediate part on both or either one of main surfaces including one main surface and another main surface of the belt part main body, and the serration part is disposed so as to be opposed to the deformed nail surface when the first latching part is latched at one end edge of the deformed nail in the width direction.

3. The nail deformity correction device according to claim 1 or 2, wherein
the serration part includes a sawtooth part having an upright surface extending at a substantially right angle with respect to the one main surface and the other main surface of the belt part and a tilted surface tilted toward the inserting direction of the belt part, and
the lock nail part includes
a nail piece having an upright engaging surface to be engaged with the upright surface of the sawtooth part and a tilted engaging surface to be engaged with the tilted surface of the sawtooth part.

4. The nail deformity correction device according to any one of claims 1 to 3, wherein
the lock nail part is disposed inside the fastening locking part via a cantilever-like movable piece.

5. The nail deformity correction device according to any one of claims 1 to 4, wherein
the movable piece includes a hinge part, and the lock nail part is supported so as to freely rock to a direction orthogonal to the inserting direction of the belt part by the hinge part.

6. The nail deformity correction device according to any one of claims 3 to 5, wherein
when an angle at which the upright surface and the tilted surface of the sawtooth part cross is taken as θ_{B}, the θ_{B} is set so that 45°<θ_{B}<60°,
when an angle at which the upright engaging surface and the tilted engaging surface of the lock nail part cross is taken as θ_{L}, the θ_{L} is set so that 45°<θ_{L}<60°, and, furthermore,
it is set so that the θ_{B}=the θ_{L}.

7. The nail deformity correction device according to any one of claims 1 or 2, wherein
the fastening locking part further includes an activation piece connected to the lock nail part and exposed to outside the fastening locking part, and
the activation piece releases a locked state between the serration part and the lock nail part by making the activation piece movable in a direction orthogonal to the inserting direction of the belt part and releasing locking between the serration part and the lock nail part.

8. The nail deformity correction device according to any one of claims 3 to 6, wherein
the fastening locking part further includes an activation piece connected to the lock nail part and exposed to outside the fastening locking part, and
the activation piece releases an engaged state between the upright surface of the sawtooth part and the upright engaging surface of the lock nail part and an engaged state between the tilted surface of the sawtooth part and the engaging tilted surface of the lock nail part by making the activation piece movable in a direction orthogonal to the inserting direction of the belt part and isolating an engagement between the sawtooth part and the lock nail part.

9. The nail deformity correction device according to any one of claims 1 to 8, wherein
side wall parts are disposed on both side surface of the belt part in a width direction along the length direction of the belt part where at least the serration part is disposed, and
a constricted part constricted to the width direction of the belt part is formed partially on each of the side wall parts so as to be opposed to each other in the width direction of the belt part.

10. The nail deformity correction device according to any one of claims 2 to 9, wherein
the lead part of the belt part is formed so as to be in a tapered shape and have a thin cross section.

11. The nail deformity correction device according to any one of claims 2 to 10, wherein
the first latching part includes a first insert part supported by the first support part,
the fastening locking part further includes a second support part provided to extend from an end edge part of the fastening locking part on an exit side of the insertion hole to the inserting direction of the belt part, and
the second latching part includes a second insert part supported by the second support part.

12. The nail deformity correction device according to any one of claims 1 to 11, wherein
the belt part and at least the lock nail part of the fastening locking part of the second straightening member are formed of a flexible plastic material, and
the first latching part and the second latching part are each formed of an elastic metal material.
